(19) Europäisches Patentamt
European Patent Office
Office européen des brevets

(11) EP 1 590 673 B1

(12) # EUROPEAN PATENT SPECIFICATION

(45) Date of publication and mention of the grant of the patent:
**15.04.2009 Bulletin 2009/16**

(21) Application number: **04707222.8**

(22) Date of filing: **02.02.2004**

(51) Int Cl.:
***G01N 33/68*** *(2006.01)*

(86) International application number:
**PCT/EP2004/000916**

(87) International publication number:
**WO 2004/070388 (19.08.2004 Gazette 2004/34)**

(54) **DIAGNOSTIC USE OF SCN2B FOR ALZHEIMER'S DISEASE**

DIAGNOSTISCHE VERWENDUNG VON SCN2B FÜR ALZHEIMER ERKRANKUNG

UTILISATION DIAGNOSTIQUE DE SCN2B POUR LA MALADIE D'ALZHEIMER

(84) Designated Contracting States:
**AT BE BG CH CY CZ DE DK EE ES FI FR GB GR HU IE IT LI LU MC NL PT RO SE SI SK TR**

(30) Priority: **04.02.2003 US 444666 P**

(43) Date of publication of application:
**02.11.2005 Bulletin 2005/44**

(73) Proprietor: **EVOTEC Neurosciences GmbH
22525 Hamburg (DE)**

(72) Inventors:
- **VON DER KAMMER, Heinz
22607 Hamburg (DE)**
- **POHLNER, Johannes
22175 Hamburg (DE)**
- **HANES, Jozef
84107 Bratislava (SK)**

(74) Representative: **Meyers, Hans-Wilhelm
Patentanwälte
von Kreisler-Selting-Werner
Postfach 10 22 41
50462 Köln (DE)**

(56) References cited:
**WO-A-00/12711**
**WO-A-02/084297**
**WO-A-03/060525**
**US-A1- 2002 127 584**

- **DWORAKOWSKA B ET AL: "ION CHANNELS-RELATED DISEASES" ACTA BIOCHIMICA POLONICA, vol. 47, no. 3, 2000, pages 685-703, XP001022116 ISSN: 0001-527X**
- **JARVIS AND ZAMPONI: "Trafficking and regulation of nezral voltage-gated calcium channels" CURRENT OPINION IN CELL BIOLOGY, vol. 19, 2007, pages 474-482,**
- **ANGLIDES ET AL: "Distribution and lateral mobility of voltage-dependent sodium channels in neurons" THE JOURNAL OF CELL BIOLOGY, vol. 106, 1988, pages 1911-1925,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 590 673 B1

## Description

The present invention relates to methods of diagnosing or, prognosticating Alzheimer's diseases in a subject.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70% of all dementia cases, and it is probably the most devastating age-related neurodegenerative condition affecting about 10% of the population over 65 years of age and up to 45% over age 85 (for a recent review see Vickers et al., Progress in Neurobiology 2000, 60: 139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β (Aβ) peptide evolves from the cleavage of the amyloid precursor protein (APP) by different kinds of proteases. The cleavage by the β/γ-secretase leads to the formation of Aβ peptides of different lengths, typically a short more soluble and slow aggregating peptide consisting of 40 amino acids and a longer 42 amino acid peptide, which rapidly aggregates outside the cells, forming the characteristic amyloid plaques (Selkoe, Physiological Rev 2001, 81: 741-66; Greenfield et al., Frontiers Bioscience 2000, 5: D72-83). Two types of plaques, diffuse plaques and neuritic plaques, can be detected in the brain of AD patients, the latter ones being the classical, most prevalent type. They are primarily found in the cerebral cortex and hippocampus. The neuritic plaques have a diameter of 50μm to 200μm and are composed of insoluble fibrillar amyloids, fragments of dead neurons, of microglia and astrocytes, and other components such as neurotransmitters, apolipoprotein E, glycosaminoglycans, α1-antichymotrypsin and others. The generation of toxic Aβ deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, Acta Neuropathol 1991, 82: 239-259). NFTs emerge inside neurons and consist of chemically altered tau, which forms paired helical filaments twisted around each other. Along the formation of NFTs, a loss of neurons can be observed. It is discussed that said neuron loss may be due to a damaged microtubule-associated transport system (Johnson and Jenkins, J Alzheimers Dis 1996, 1: 38-58; Johnson and Hartigan, J Alzheimers Dis 1999, 1: 329-351). The appearance of neurofibrillary tangles and their increasing number correlates well with the clinical severity of AD (Schmitt et al., Neurology 2000, 55: 370-376).

AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92).

The age of onset of AD may vary within a range of 50 years, with early-onset AD occurring in people younger than 65 years of age, and late-onset of AD occurring in those older than 65 years. About 10% of all AD cases suffer from early-onset AD, with only 1-2% being familial, inherited cases.

Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977-81; Roses, Ann NY Acad Sci 1998, 855: 738-43). The polymorphic plasmaprotein ApoE plays a role in the intercellular cholesterol and phospholipid transport by binding low-density lipoprotein receptors, and it seems to play a role in neurite growth and regeneration. Efforts to detect further susceptibility genes and disease-linked polymorphisms, lead to the assumption that specific regions and genes on human chromosomes 10 and 12 may be associated with late-onset AD (Myers et al., Science 2000, 290: 2304-5; Bertram et al., Science 2000, 290: 2303; Scott et al., Am J Hum Genet 2000, 66: 922-32).

Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide insight into the pathogenesis of neurological diseases and to provide methods, materials, agents, compositions, and non-human animal models which are suited inter alia for the diagnosis and development of a treatment of these diseases. This object has been solved by the features of the independent claims. The subclaims define preferred embodiments of the present invention.

The present invention is based on the differential expression of a gene coding for the voltage-gated sodium channel type II beta-subunit (beta-2 subunit, β2-subunit, β2 or SCN2B) in human Alzheimer's disease brain samples. Consequently, the SCN2B gene and its corresponding transcription and/or translation products may have a causative role in the regional selective neuronal degeneration typically observed in AD. Based on these disclosures, the present invention has utility for the diagnostic evaluation and prognosis as well as for the identification of a predisposition to AD.

Voltage-gated ion channels play an important role in the nervous system by generating conducted action potentials. Ion-conducting membrane channels for cations (sodium, calcium, potassium) and anions (chloride) are described (Lehmann-Horn et al., Physiological Reviews 1999, 79: 1317-1358). Transport of ions across the cell membrane leads to a fast transmission of electrical impulses throughout the cell network. Thereby, the channel switches between three functionally distinct states: a resting, an active, and an inactive one. Both, the resting and inactive states are nonconducting, and the channel is closed. As the membrane potential increases from less than -60 mV, the channel starts to open its pore (i.e. activation). Influx of ions (e.g. sodium) leads to a further increase of the membrane potential until an action potential is initiated. By closing the pore within 1 millisecond (i.e. fast inactivation) or within seconds to minutes (i.e. slow inactivation), the channel rapidly returns to an inactivated state. The ion conductance is highly selective and efficient which enables fine tuning of processes such as memory, movement, and cognition (Lehmann-Horn et al., Physiological Reviews 1999, 79: 1317-1358). Molecular cloning of voltage-gated ion channels has uncovered a diversity of subtypes and enhanced the understanding about the underlying structure and function, particularly of sodium channels (Noda et al., Nature 1986, 322: 826-828; Schaller et al., Journal of Neuroscience 1995, 15: 3231-3242; Isom et al., Neuron 1994, 12: 1183-1194; Isom et al., Cell 1995, 83: 443-445).

Voltage-gated sodium channels are composed of a pore-forming α-subunit with approximately 260 kDa and of two smaller auxiliary β-subunits, β1 with ~36kDa and β2 with ~33kDa, which form a heteromeric complex. The α-subunit and both β-subunits are highly glycosylated (deglycosylated subunits with ~220 kDa, ~23 kDa, ~21 kDa for α, β1 and β2, respectively). Three different genes encoding β-subunits have been identified to date. The β1-subunit is noncovalently associated with the α-subunit, whereas the β2-subunit is covalently attached to the α-subunit via a disulfide bridge. A third β-subunit isoform (β3), similar to the β1-subunit, has recently been discovered (Morgan et al., Proceedings National Academy of Science USA 2000, 97: 2308-2313). The α-subunit appears to be necessary and sufficient for sodium channel functionality. The β-subunit modulate sodium channel function by accelerating the rate of inactivation, by increasing the amplitude $Na^+$ peak current, by increasing the activation rate and by altering voltage dependency (Patton et al., Journal Biological Chemistry 1994, 269: 17649-17655). The β2-subunits appear not to be as effective as the β1-subunits, thus both may cooperate in modulating the function of the α-subunit.

The human β2-subunit is a glycoprotein with a signal peptide sequence of 29 amino acids, which is cleaved off during maturation of the protein. Additionally, the protein is made up of a short intracellular C-terminal segment, a single transmembrane region, and a large extracellular domain at the amino terminal, comprising conserved cysteine residues which are able to form a disulfide linkage with the α-subunit. The β-subunits exhibit an immunoglobulin-like motif, i.e. immunoglobulin-like C2-type domain, with structural similarities to cell adhesion molecules (CAM), for example contactin. Therefore, the β2-subunit might interact with extracellular matrix proteins such as tenascin-C and tenascin-R (Isom et al., Cell 1995, 83: 443-445). Cell adhesion molecules, such as the neuronal CAM (NCAM) and the myelin-associated glycoprotein (MAG), have immunoglobulin folds of the same type as the β2-subunit. The β2-subunit differ from the other β-subunits in that they feature a CAM motif and in the ability to locally expand the cell surface membrane. Thus, besides being modulators for sodium channels, they may function in cell-cell interaction, as regulators for sodium channel expression, and may regulate localization and immobilization of sodium channels in specific regions of the plasma membrane. Human β2 has similarity to myelin protein zero (p0), a transmembrane glycoprotein and a major structural component of peripheral myelin (Eubanks et al., Neuroreport 1997, 8: 2775-2779).

The genomic structure of the SCN2B gene was reported in 1998 (GenBank Accession No. AF049496; AF049497; Bolino et al., European Journal of Human Genetics 1998, 6: 629-634; and GenBank Accession No. AF049498; complete coding sequence and corresponding protein sequence). Mapping studies locate SCN2B to chromosome 11 q23 (Eubanks et al., Neuroreport 1997, 8: 2775-2779). SCN2B covers approximately 12 kb of genomic DNA, harboring 4 exons and 3 introns which encode the mature SCN2B protein of 186 amino acids, and the SCN2B protein of 215 amino acids including the putative signal peptide of 29 amino acids (protein sequence and putative domains: GenBank Accession No. 060939). The human and rat SCN2B proteins are of equal length and highly conserved (89% homology and 93% similarity).

Four brain sodium channels, i.e. SCN1A, SCN2A, SCN3A, and SCN8A, exhibit major expression in the central nervous system. The mRNAs of the β1 and β2-subunits (transcripts of -2 kb and ~4 kb, respectively) have a broad but distinct regional expression pattern. In contrast to the β1-subunit encoding SCN1B gene, the SCN2B gene is expressed in the central nervous system only. Predominant expression levels were observed in the parahippocampal gyrus, the cortex, the granular layer, and Purkinje cells of the cerebellum. In rat brain, expression of β2 starts with embryonic day 9, in parallel with neurogenesis, and rapidly increases with axon extension and synaptogenesis (Isom et al.; Cell 1995, 83: 443-445).

For the neuronal sodium channel subunit genes SCN1A and SCN1B, disease-causing mutations leading to generalized epilepsy with febrile seizures plus (GEFS+) have been identified. Therefore, it is speculated that variations in the SCN2B gene may also result in common subtypes of idiopathic generalized epilepsy. Haug and coworkers identified a novel single nucleotide polymorphism (SNP) and a missense mutation (Asn209Pro) in the SCN2B gene, but excluded the SCN2B gene as a major susceptibility gene in epilepsy (Haug et al., Neuroreport 2000, 11: 2687-2689). Patent applications WO 02/19966, WO 02/087419, and WO 02/090532 implicate sodium channel β-subtypes with cardiovascular, inflammatory and muscular diseases, cancer and epilepsy. On the basis of protein homology and chromosomal localization, SCN2B has also been considered a suitable candidate gene for the demyelinating autosomal recessive motor and sensory neuropathy called Charcot-Marie-Tooth disease (CMT4B). This notion, however, was disputed by Bolino and coworkers (Bolino et al., European Journal of Human Genetics 1998, 6: 629-634). Nonetheless, patent application WO 01/79547 describes single nucleotide polymorphisms in the SCN2B gene which might be associated with demyelinating disorders. To date, there are no reports on a relationship between the β2-subunit of the voltage-gated ion channel type II and neurodegenerative disorders such as Alzheimer's disease.

Sodium channels are valuable targets for a variety of drugs as local anesthetics, anticonvulsants, antiarrythmics, for the treatment of neuropathic pain, epilepsy, and stroke. A number of toxins, drugs, and inorganic cations are used by the pharmaceutical industry as blockers of the α-subunit of sodium channels in central nervous system related disorders. Inhibitors of voltage-gated ion channels are already on the market, albeit they are of low potency, relatively non-specific, and their therapeutic potential is not fully exploited. Remarkably, no drugs are currently known that interact with the β-subunits of sodium channels. Thus, it is desirable to find specific drugs for a selective subunit of voltage-gated sodium channels such as the β2-subunit.

The present invention discloses a dysregulation of voltage-gated sodium channel beta-2 subunit (β2-subunit, SCN2B) gene expression in brain samples from Alzheimer's disease patients. No such dysregulation is observed in samples derived from age-matched, healthy controls. To date, no experiments have been described that demonstrate a relationship between the dysregulation of β2-subunit gene expression and the pathology of Alzheimer's disease. Likewise, no mutations in the β2-subunit gene have been described to be associated with said diseases. Linking the β2-subunit gene to Alzheimer's disease, as disclosed in the instant invention, offers new ways, inter alia, for the diagnosis of said diseases.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity. The terms "level" and/or "activity" as used herein further refer to gene expression levels or gene activity. Gene expression can be defined as the utilization of the information contained in a gene by transcription and translation leading to the production of a gene product. "Dysregulation" shall mean an upregulation or downregulation of gene expression. A gene product comprises either RNA or protein and is the result of expression of a gene. The amount of a gene product can be used to measure how active a gene is. The term "gene" as used in the present specification and in the claims comprises both coding regions (exons) as well as non-coding regions (e.g. non-coding regulatory elements such as promoters or enhancers, introns, leader and trailer sequences). The term "ORF" is an acronym for "open reading frame" and refers to a nucleic acid sequence that does not possess a stop codon in at least one reading frame and therefore can potentially be translated into a sequence of amino acids. "Regulatory elements" shall comprise inducible and non-inducible promoters, enhancers, operators, and other elements that drive and regulate gene expression. The term "fragment" as used herein is meant to comprise e.g. an alternatively spliced, or truncated, or otherwise cleaved transcription product or translation product. The terms "agent", "reagent", or "compound" refer to any substance, chemical, composition or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. As used herein, "homolog or homology" is a term used in the art to describe the relatedness of a nucleotide or peptide sequence to another nucleotide or peptide sequence, which is determined by the degree of identity and/or similarity between said sequences compared. In the art, the terms "identity" and "similarity" mean the degree of polypeptide or polynucleotide sequence relatedness which are determined by matching a query sequence and other sequences of preferably the same type (nucleic acid or protein sequence) with each other. Preferred computer program methods to calculate and determine "identity" and "similarity" include, but are not limited to GCG BLAST (Basic Local Alignment Search Tool) (Altschul et al., J. Mol. Biol. 1990, 215: 403-410; Altschul et al., Nucleic Acids Res. 1997, 25: 3389-3402; Devereux et al., Nucleic Acids Res. 1984, 12: 387), BLASTN 2.0 (Gish W., 1996-2002), FASTA (Pearson and Lipman, Proc. Natl. Acad. Sci. USA 1988, 85: 2444-2448), and GCG GelMerge which determines and aligns a pair of contigs with the longest overlap (Wilbur and Lipman, SIAM J. Appl. Math. 1984, 44: 557-567; Needleman and Wunsch, J. Mol. Biol. 1970, 48: 443-453). The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention, but retains its essential properties. "Proteins and polypeptides" of the present invention include variants, fragments and chemical derivatives of the protein comprising the amino acid sequences of SCN2B, SEQ ID NO. 1. They can include proteins and polypeptides which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants. The term "isolated" as used herein is considered to refer to molecules or substances which have been changed and/or which have been removed from their natural environment, i.e. isolated from a cell or from a living organism in which they normally occur, and that are separated or essentially purified from the coexisting components with which they are found to be associated in nature. This notion further means that the sequences encoding such molecules can be linked by the hand of man to polynucleotides, to which they are not linked in their natural state, and that such molecules can be produced by recombinant and/or synthetic means. Even if for said purposes those sequences may be introduced into living or non-living organisms by methods known to those skilled in the art, and even if those sequences are still present in said organisms, they are still considered to be isolated. In the present invention, the terms "risk", "susceptibility", and "predisposition" are tantamount and are used with respect to the probability of developing Alzheimer's disease.

The term 'AD' shall mean Alzheimer's disease. "AD-type neuropathology" as used herein refers to neuropathological, neurophysiological, histopathological and clinical hallmarks as described in the instant invention and as commonly known from state-of-the-art literature (see: Iqbal, Swaab, Winblad and Wisniewski, *Alzheimer's Disease and Related Disorders (Etiology, Pathogenesis and Therapeutics),* Wiley & Sons, New York, Weinheim, Toronto, 1999; Scinto and Daffner, *Early Diagnosis of Alzheimer's Disease,* Humana Press, Totowa, New Jersey, 2000; Mayeux and Christen, *Epidemiology of Alzheimer's Disease: From Gene to Prevention,* Springer Press, Berlin, Heidelberg, New York, 1999; Younkin, Tanzi and Christen, *Presenilins and Alzheimer's Disease,* Springer Press, Berlin, Heidelberg, New York, 1998).

Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, ischemic stroke, age-related macular degeneration, narcolepsy, motor neuron diseases, prion diseases, traumatic nerve injury and repair, and multiple sclerosis.

In one aspect, the invention features a method of diagnosing or prognosticating Alzheimer's disease in a subject, or determining whether a subject is at increased risk of developing said disease. The method comprises: determining a level, or an activity, or both said level and said activity of (i) a transcription product of the gene coding for SCN2B, and/or of (ii) a translation product of the gene coding for SCN2B, and/or of (iii) a fragment of said transcription or translation product in a brain sample from said subject and comparing said level, and/or said activity to a reference value representing a known disease or health status, thereby diagnosing or prognosticating Alzheimer's disease in said subject, or determining whether said subject is at increased risk of developing said disease if SCN2B is dysregulated.

The invention also relates to the construction and the use of primers and probes which are unique to the nucleic acid sequences, or fragments, thereof, as disclosed in the present invention. The oligonucleotide primers and/or probes can be labeled specifically with fluorescent, bioluminescent, magnetic, or radioactive substances. The invention further relates to the detection and the production of said nucleic acid sequences, or fragments and variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects. Whether an amplification results in a specific nucleic acid product or not, and whether a fragment of different length can be obtained or not, may be indicative for Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with Alzheimer's disease. This feature has utility for developing rapid DNA-based diagnostic tests, preferably also in the format of a kit.

In a preferred embodiment of the herein claimed and uses of the instant invention, said gene coding for a human voltage-gated sodium channel beta subunit protein is the gene coding for a human voltage-gated sodium channel type II beta subunit (SCN2B), also termed beta-2 subunit protein or β2-protein represented by the gene coding for the protein of Genbank accession number 060939 (protein ID), SEQ ID NO. 1, which is deduced from the mRNA corresponding to the cDNA sequences of Genbank accession numbers AF049498, AF049496, AF049497, AF107028, AX376000 and ESTs from the Genbank database, SEQ ID NO. 2. In the instant invention SCN2B also refers to the nucleic acid sequence of SEQ ID NO. 2, coding for the protein of SEQ ID NO. 1 (Genbank accession number 060939). In the instant invention said sequences are "isolated" as the term is employed herein. Further, in the instant invention, the gene coding for said SCN2B protein is also generally referred to as the SCN2B gene, or simply SCN2B, and the protein of SCN2B is also generally referred to as the SCN2B protein, or simply SCN2B.

The present invention discloses the detection and differential expression and regulation of the gene coding for SCN2B in specific brain regions of AD patients.

Consequently, the SCN2B gene and its corresponding transcription and/or translation products may have a causative role in the regional selective neuronal degeneration typically observed in AD. Alternatively, SCN2B may confer a neuroprotective function to the remaining surviving nerve cells. Based on these disclosures, the present invention has utility for the diagnostic evaluation and prognosis as well as for the identification of a predisposition to AD.

It is preferred that the sample to be analyzed and determined is selected from the group comprising brain tissue, or other tissues, or body cells.

In further preferred embodiments, said reference value is that of a level, or an activity, or both said level and said activity of (i) a transcription product of the gene coding for SCN2B, and/or of (ii) a translation product of the gene coding for SCN2B, and/or of (iii) a fragment of said transcription or translation product in a brain sample from a subject not suffering from said Alzheimer's disease.

In preferred embodiments, an alteration in the level and/or activity of a transcription product of the gene coding for SCN2B and/or of a translation product of the gene coding for SCN2B and/or of a fragment thereof in a brain sample from said subject relative to a reference value representing a known health status indicates a diagnosis, or prognosis, or increased risk of becoming diseased with AD.

In preferred embodiments, measurement of the level of transcription products of the SCN2B gene is performed in a brain sample from a subject using a quantitative PCR-analysis with primer combinations to amplify said gene specific sequences from cDNA obtained by reverse transcription of RNA extracted from a brain sample of a subject. A Northern blot with probes specific for said gene can also be applied. It might further be preferred to measure transcription products by means of chip-based microarray technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO 02/14543.

Furthermore, a level and/or an activity of a translation product of the gene coding for SCN2B and/or of a fragment of said translation product, and/or the level of activity of said translation product, and/or of a fragment thereof, can be detected using an immunoassay, an activity assay, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots, and other techniques known to those of ordinary skill in the art (see Harlow and Lane, Using Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip based technologies (see Schena M., Microarray Biochip Technology, Eaton Publishing, Natick, MA, 2000).

In a preferred embodiment, the level, or the activity, or both said level and said activity of (i) a transcription product of the gene coding for SCN2B, and/or of (ii) a translation product of the gene coding for SCN2B, and/or of (iii) a fragment of said transcription or translation product in a series of brain samples taken from said subject over a period of time is compared, in order to monitor the progression of Alzheimer's disease.

The present invention features the use of an antibody which is specifically immunoreactive with a translation product of the gene coding for SCN2B, SEQ ID NO.1, or a fragment thereof. Methods for generating antibodies are well known in the art (see Harlow et al., Antibodies: A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies of the present invention are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme-immuno assays (e.g. enzyme-linked immunosorbent assay, ELISA); radioimmuno assays, chemoluminescence-immuno assays, Western-blot, immunoprecipitation and antibody microarrays. These methods involve the detection of translation products of the SCN2B gene, or fragments thereof.

Said antibodies are used for detecting the pathological state of a cell in a brain sample from a subject, comprising immunocytochemical staining of said cell, wherein an altered degree of staining in said cell compared to a cell representing a known health status indicates a pathological state of said cell and wherein the pathological state relates to AD. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

Other features and advantages of the invention will be apparent from the following description of figures and examples which are illustrative only and not intended to limit the remainder of the disclosure in any way.

Figure 1 depicts the brain regions with selective vulnerability to neuronal loss and degeneration in AD. Primarily, neurons within the inferior temporal lobe, the entorhinal cortex, the hippocampus, and the amygdala are subject to degenerative processes in AD (Terry et al., Annals of Neurology 1981, 10: 184-192). These brain regions are mostly involved in the processing of learning and memory functions. In contrast, neurons within the frontal cortex, the occipital cortex, and the cerebellum remain largely intact and preserved from neurodegenerative processes in AD. Brain tissues from the frontal cortex (F), the temporal cortex (T) and the hippocampus (H) of AD patients and healthy, age-matched control individuals were used for the herein disclosed examples. For illustrative purposes, the image of a normal healthy brain was taken from a publication by Strange (Brain Biochemistry and Brain Disorders, Oxford University Press, Oxford, 1992, p.4).

Figure 2 discloses the initial identification of the differential expression of the gene coding for SCN2B in a fluorescence differential display screen. The figure shows a clipping of a large preparative fluorescent differential display gel. PCR products from the frontal cortex (F) and the temporal cortex (T) of two healthy control subjects and six AD patients were loaded in duplicate onto a denaturing polyacrylamide gel (from left to right). PCR products were obtained by amplification of the individual cDNAs with the corresponding one-base-anchor oligonucleotide and the specific Cy3 labelled random primers. The arrow indicates the migration position where significant differences in intensity of the signals for a transcription product of the gene coding for SCN2B derived from frontal cortex as compared to the signals derived from the temporal cortex of AD patients exist. The differential expression reflects a down-regulation of SCN2B gene transcription in the temporal cortex compared to the frontal cortex of AD patients. Comparing the signals derived from temporal cortex and frontal cortex of healthy non-AD control subjects with each other, no difference in signal intensity, i.e. no altered expression level can be detected.

Figure 3 and Figure 4 illustrate the verification of the differential expression of the human SCN2B gene in AD brain tissues by quantitative RT-PCR analysis using two different set of primer pairs, primer set A (Figure 3) and primer set B (Figure 4). Quantification of RT-PCR products from RNA samples collected from the frontal cortex (F) and temporal cortex (T) of AD patients (Figure 3a; 4a) and of a healthy, age-matched control individuals (Figure 3b; 4b) was performed by the LightCycler rapid thermal cycling technique. The data were normalized to the combined average values of a set of standard genes which showed no significant differences in their gene expression levels. Said set of standard genes consisted of genes for the ribosomal protein S9, the transferrin receptor, GAPDH, cyclophilin B, and beta-actin. The figures depict the kinetics of amplification by plotting the cycle number against the amount of amplified material as measured by its fluorescence. Note that the amplification kinetics of the SCN2B cDNA from both the frontal and temporal cortices of a normal control individual during the exponential phase of the reaction are juxtaposed (Figure 3b; 4b: arrows), whereas in AD (Figure 3a; 4a: arrows), there is a significant separation of the corresponding curves, indicating a differential expression of the SCN2B gene in the two analyzed brain regions.

Figure 5 illustrates the differential expression of the human SCN2B gene in AD brain tissues by quantitative RT-PCR analysis using primer set A. Quantification of RT-PCR products from RNA samples collected from the frontal cortex (F) and from the hippocampus (H) of AD patients (Figure 5a) was performed by the LightCycler rapid thermal cycling technique. Likewise, samples of healthy, age-matched control individuals were compared (Figure 5b). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figures depict the kinetics of amplification by plotting the cycle number against the amount of amplified material as measured by its fluorescence. Note that the amplification kinetics of SCN2B cDNAs from the frontal cortex and the hippocampus of a normal control individual during the exponential phase of the reaction are juxtaposed (Figure 5b, arrowheads), whereas in Alzheimer's disease (Figures 5a, arrowheads) there is a significant separation of the corresponding curves, indicating a differential expression of the gene coding for SCN2B in the respective analyzed brain regions, preferably a dysregulation of a transcription product of the human SCN2B gene, or a fragment, or derivative, or variant thereof, in the frontal cortex relative to the hippocampus.

Figure 6 discloses SEQ ID NO. 1; the amino acid sequence of the SCN2B protein. The full length human SCN2B protein comprises 215 amino acids.

Figure 7 shows SEQ ID NO. 2, the nucleotide sequence of the human SCN2B cDNA, comprising 4903 nucleotides, represented by a consensus sequence resulting from the assembly of a large redundant set of overlapping human nucleotide sequence fragments found in the Genbank human genomic database (see Figure 10).

Figure 8 depicts SEQ ID NO. 3, the nucleotide sequence of the 99 bp SCN2B cDNA fragment, identified and obtained by differential display (sequence in 5' to 3' direction).

Figure 9 outlines the sequence alignment of SEQ ID NO. 3 to the nucleotide sequence of the human SCN2B cDNA, SEQ ID NO. 2.

Figure 10 schematically charts the assembly of SEQ ID NO. 2 from genomic database sequence fragments, constituting the SCN2B consensus cDNA sequence, a prolongated and corrected consensus sequence based on and derived from GenBank accession numbers AF049498, AF107028, AX376000, HSU87555 and the other indicated EST sequence fragments, and sequences derived from PCR amplification (ens-PCR1, ens-PCR2) with primers for the gene coding for SCN2B. Primer Set D. (ens-PCR1, sequence nucleotides 3204 to 4182 of SEQ ID NO. 2): 5'-TGAGCGAGTCAAGCCCATCTGG-3' and 5'-CCAAAGCCAGTTCCAAGGCACCTC-3'; Primer Set C (ens-PCR2, sequence nucleotides 2138 to 3230 of SEQ ID NO. 2): 5'-GGGAAGCAGAGGTTGCAGTGAAC-3' and 5'-CATTTCCAGATGGGCTTGACTCGC TC-3'.

Figure 11 shows a schematic alignment of SEQ ID NO. 3 with SCN2B cDNA. The open rectangle represents the SCN2B open reading frame, thin bars represent the 5' and 3' untranslated regions (UTRs).

Figure 12 depicts sections from human pre-central gyrus labeled with an affinity-purified rabbit polyclonal anti-SCN2B antiserum (green signals) raised against a peptide corresponding to amino acids 179 to 215 of SCN2B. Immunoreactivity of SCN2B was observed in both the cerebral cortex (CT) and the white matter (WM) (Figure 12a, low magnification). A punctate pattern of SCN2B immunoreactivity decorating neuronal processes (dendritic spine-like) and a weaker and diffuse immunoreactivity of neuronal somata were observed (Figure 12b, high magnification). Figure 12c shows punctate immunoreactivity of glial cell bodies and nerve fibers. Blue signals indicate nuclei stained with DAPI.

The table in figure 13 lists SCN2B gene expression levels in the temporal cortex relative to the frontal cortex in fifteen AD patients, herein identified by internal reference numbers P010, P011, P012, P014, P016, P017, P019, P026, P031, P038, P040, P041, P042, P046, P047 (0.29 to 0.98 fold) and nineteen healthy, age-matched control individuals, herein identified by internal reference numbers C005, C008, C011, C012, C014, C020, C022, C024, C025, C026, C027, C028, C029, C030, C031, C032, C033, C034, C035 (0.59 to 2.15 fold). Quantitative RT-PCR analysis was performed using primer pair set A. The values shown are calculated according to the formula described herein (see below). The scatter diagram visualizes individual logarithmic values of the temporal to frontal cortex regulation ratios, log(ratio IT/IF), in control samples (dots) and in AD patient samples (triangles), respectively.

The table in figure 14 lists SCN2B gene expression levels in the temporal cortex relative to the frontal cortex in seven AD patients, herein identified by internal reference numbers P010, P011, P012, P014, P016, P017, P019 (0.20 to 0.91 fold) and five healthy, age-matched control individuals, herein identified by internal reference numbers C005, C008, C011, C012, C014 (0.48 to 1.48 fold). Quantitative RT-PCR analysis was performed using primer pair set B. The values shown are calculated according to the formula described herein (see below). The scatter diagram visualizes individual logarithmic values of the temporal to frontal cortex regulation ratios, log(ratio IT/IF), in control samples (dots) and in AD patient samples (triangles), respectively.

The table in figure 15 lists the gene expression levels in the hippocampus relative to the frontal cortex for the SCN2B gene in six Alzheimer's disease patients, herein identified by internal reference numbers P010, P011, P012, P014, P016, P019 (0.48 to 10.87 fold) and three healthy, age-matched control individuals, herein identified by internal reference numbers C004, C005, C008 (0.77 to 1.26 fold). Quantitative RT-PCR analysis was performed using primer pair set A. The values shown are calculated according to the formula described herein (see below). The scatter diagram visualizes individual logarithmic values of the hippocampus to frontal cortex regulation ratios, log(ratio HC/IF), in control samples (dots) and in AD patient samples (triangles).

EXAMPLE I:

(i) Brain tissue dissection from patients with AD:

Brain tissues from AD patients and age-matched control subjects were collected within 6 hours post-mortem and immediately frozen on dry ice. Sample sections from each tissue were fixed in paraformaldehyde for histopathological confirmation of the diagnosis. Brain areas for differential expression analysis were identified (see Figure 1) and stored at -80°C until RNA extractions were performed.

(ii) Isolation of total mRNA:

Total RNA was extracted from post-mortem brain tissue by using the RNeasy kit (Qiagen) according to the manufacturer's protocol. The accurate RNA concentration and the RNA quality were determined with the DNA LabChip system using the Agilent 2100 Bioanalyzer (Agilent Technologies). For additional quality testing of the prepared RNA, i.e. exclusion of partial degradation and testing for DNA contamination, specifically designed intronic GAPDH oligonucleotides and genomic DNA as reference control were used to generate a melting curve with the LightCycler technology as described in the manufacturer's protocol (Roche).

(iii) cDNA synthesis and identification of differentially expressed genes by fluorescence differential display (FDD):

In order to identify changes in gene expression in different tissues we employed a modified and improved differential display (DD) screening method. The original DD screening method is known to those skilled in the art (Liang and Pardee, Science 1995, 267:1186-7). This technique compares two populations of RNA and provides clones of genes that are expressed in one population but not in the other. Several samples can be analyzed simultaneously and both up- and down-regulated genes can be identified in the same experiment. By adjusting and refining several steps in the DD method as well as modifying technical parameters, e.g. increasing redundancy, evaluating optimized reagents and conditions for reverse transcription of total RNA, optimizing polymerase chain reactions (PCR) and separation of the products thereof, a technique was developed which allows for highly reproducible and sensitive results. The applied and improved DD technique was described in detail by von der Kammer et al. (Nucleic Acids Research 1999, 27: 2211-2218). A set of 64 specifically designed random primers were developed (standard set) to achieve a statistically comprehensive analysis of all possible RNA species. Further, the method was modified to generate a preparative DD slab-gel technique, based on the use of fluorescently labelled primers. In the present invention, RNA populations from carefully selected post-mortem brain tissues (frontal and temporal cortex) of Alzheimer's disease patients and age-matched control subjects were compared.
As starting material for the DD analysis we used total RNA, extracted as described above (ii). Equal amounts of 0.05 µg RNA each were transcribed into cDNA in 20 µl reactions containing 0.5 mM each dNTP, 1 µl Sensiscript™ Reverse Transcriptase and 1x RT buffer (Qiagen), 10 U RNase inhibitor (Qiagen) and 1 µM of either one-base-anchor oligonucleotides $HT_{11}A$, $HT_{11}G$ or $HT_{11}C$ (Liang et al., Nucleic Acids Research 1994, 22: 5763-5764; Zhao et al., Biotechniques 1995, 18:842-850).

Reverse transcription was performed for 60 min at 37 °C with a final denaturation step at 93 °C for 5 min. 2 µl of the obtained cDNA each was subjected to a polymerase chain reaction (PCR) employing the corresponding one-base-anchor oligonucleotide (1 µM) along with either one of the Cy3 labelled random DD primers (1 µM), 1x GeneAmp PCR buffer (Applied Biosystems), 1.5 mM $MgCl_2$ (Applied Biosystems), 2 µM dNTP-Mix (dATP, dGTP, dCTP, dTTP Amersham Pharmacia Biotech), 5 % DMSO (Sigma), 1 U AmpliTaq DNA Polymerase (Applied Biosystems) in a 20 µl final volume. PCR conditions were set as follows: one round at 94 °C for 30 sec for denaturing, cooling 1 °C/sec down to 40 °C, 40 °C for 4 min for low-stringency annealing of primer, heating 1 °C/sec up to 72 °C, 72 °C for 1 min for extension. This round was followed by 39 high-stringency cycles: 94 °C for 30 sec, cooling 1 °C/sec down to 60 °C, 60 °C for 2 min, heating 1 °C/sec up to 72 °C, 72 °C for 1 min. One final step at 72 °C for 5 min was added to the last cycle (PCR cycler: Multi Cycler PTC 200, MJ Research). 8 μl DNA loading buffer were added to the 20 μl PCR product preparation, denatured for 5 min and kept on ice until loading onto a gel. 3.5 μl each were separated on 0.4 mm thick, 6 %-polyacrylamide (Long Ranger)/ 7 M urea sequencing gels in a slab-gel system (Hitachi Genetic Systems) at 2000 V, 60W, 30 mA, for 1 h 40 min. Following completion of the electrophoresis, gels were scanned with a FMBIO II fluorescence-scanner (Hitachi Genetic Systems), using the appropriate FMBIO II Analysis 8.0 software. A full-scale picture was printed, differentially expressed bands marked, excised from the gel, transferred into 1.5 ml containers, overlayed with 200 μl sterile water and kept at -20°C until extraction.
Elution and reamplification of differential display products: The differential bands were extracted from the gel by boiling in 200 μl $H_2O$ for 10 min, cooling down on ice and precipitation from the supernatant fluids by using ethanol (Merck) and glycogen/sodium acetate (Merck) at -20 °C over night, and subsequent centrifugation at 13.000 rpm for 25 min at 4 °C. Pellets were washed twice in ice-cold ethanol (80%), resuspended in 10 mM Tris pH 8.3 (Merck) and dialysed against 10 % glycerol (Merck) for 1 h at room temperature on a 0.025 μm VSWP membrane (Millipore). The obtained preparations were used as templates for reamplification by 15 high-stringency cycles in 25-μl PCR mixtures containing the corresponding primer pairs as used for the differential display PCR (see above) under identical conditions, with the exception of the initial round at 94 °C for 5 min, followed by 15 cycles of: 94 °C for 45 sec, 60 °C for 45 sec, ramp 1 °C/sec to 70 °C for 45 sec, and one final step at 72 °C for 5 min.

Cloning and sequencing of differential display products: Re-amplified cDNAs were analyzed with the DNA LabChip system (Agilent 2100 Bioanalyzer, Agilent Technologies) and were ligated into the pCR-Blunt II-TOPO vector and transformed into E.coli Top10F' cells (Zero Blunt TOPO PCR Cloning Kit, Invitrogen) according to the manufacturer's instructions. Cloned cDNA fragments were sequenced by commercially available sequencing facilities. The results of one such fluorescence differential display experiment for the human SCN2B gene are shown in Figure 2.

(iv) Confirmation of differential expression by quantitative RT-PCR:

Positive corroboration of differential expression of the gene coding for SCN2B was performed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic, rather than an endpoint readout. The ratios of SCN2B cDNA from the temporal cortex and frontal cortex, and from the hippocampus and frontal cortex, respectively, were determined (relative quantification).

First, a standard curve was generated to determine the efficiency of the PCR with specific primers for the gene coding for SCN2B.

Primer Set A :

5'-GACTGCTGGGATGTATCTGCTTT-3' and
5'-TTGTCGCCAGTAGACCCAAAC-3'.

Primer Set B:

5'-CCAAGGCTGGGAAATGAGG-3' and
5'-CAAGGGCAACTGGGAGAGTTC-3'.

PCR amplification (95 °C and 1 sec, 56 °C and 5 sec, and 72 °C and 5 sec) was performed in a volume of 20 μl containing LightCycler-FastStart DNA Master SYBR Green I mix (contains FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and, depending on the primers used, additional 3 mM $MgCl_2$. Melting curve analysis revealed a single peak at approximately 86.5°C with no visible primer dimers. Quality and size of the PCR product were determined with the DNA LabChip system (Agilent 2100 Bioanalyzer, Agilent Technologies). A single peak at the expected size of 82 bp for primer set A and 84 bp for primer set B was observed in the electropherogram of the sample. In an analogous manner, the PCR protocol was applied to determine the PCR efficiency of a set of reference genes which were selected as a reference standard for quantification. In the present invention, the mean value of five such reference genes was determined: (1) cyclophilin B, using the specific primers 5'-ACTGAAGCACTACGGGCCTG-3' and 5'-AGCCGTTGGTGTCTT TGCC-3' except for $MgCl_2$ (an additional 1 mM was added instead of 3 mM). Melting curve analysis revealed a single peak at approximately 87 °C with no visible primer dimers. Agarose gel analysis of the PCR product showed one single band of the expected size (62 bp). (2) Ribosomal protein S9 (RPS9), using the specific primers 5'-GGTCAAATTTACCCTGGCCA-3' and 5'- TCTCATCAAGCGTCAGCAGTTC-3' (exception: additional 1 mM $MgCl_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers 5'-TGGAACGGTGAAGGTGACA-3' and 5'-GGCAAGGGACTTCCTGTAA-3'. Melting curve analysis revealed a single peak at approximately 87°C with no visible primer dimers. Agarose gel analysis of the PCR product showed one single band with the expected size (142 bp). (4) GAPDH, using the specific primers 5'-CGTCATGGGTGTGAACCATG-3' and 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers 5'-GTCGCTGGTCAGTTCGTGATT-3' and 5'-AGCAGTTGGCTGTTGTACCTCTC-3'. Melting curve analysis revealed a single peak at approximately 83°C with no visible primer dimers. Agarose gel analysis of the PCR product showed one single band with the expected size (80 bp).
For calculation of the values, first the logarithm of the cDNA concentration was plotted against the threshold cycle number $C_t$ for the gene coding for SCN2B and the five reference standard genes. The slopes and the intercepts of the standard curves (i.e. linear regressions) were calculated for all genes. In a second step, cDNAs from frontal cortex and temporal cortex, and from frontal cortex and hippocampus, respectively, were analyzed in parallel and normalized to cyclophilin B. The $C_t$ values were measured and converted to ng total brain cDNA using the corresponding standard curves:

$$10 \wedge ( (C_t \text{ value - intercept}) / \text{slope} ) \qquad [\text{ng total brain cDNA}]$$

The values for frontal and temporal cortex SCN2B cDNAs, and the values for frontal cortex and hippocampus SCN2B cDNAs, respectively, were normalized to cyclophilin B and the ratios were calculated according to formulas:

$$\text{Ratio} = \frac{\text{SCN2B temporal [ng] / cyclophilin B temporal [ng]}}{\text{SCN2B frontal [ng] / cyclophilin B frontal [ng]}}$$

$$\text{Ratio} = \frac{\text{SCN2B hippocampus [ng] / cyclophilin B hippocampus [ng]}}{\text{SCN2B frontal [ng] / cyclophilin B frontal [ng]}}$$

In a third step, the set of reference standard genes was analyzed in parallel to determine the mean average value of the temporal to frontal ratios, and of the hippocampal to frontal ratios, respectively, of expression levels of the reference standard genes for each individual brain sample. As cyclophilin B was analyzed in step 2 and step 3, and the ratio from one gene to another gene remained constant in different runs, it was possible to normalize the values for SCN2B to the mean average value of the set of reference standard genes instead of normalizing to one single gene alone. The calculation was performed by dividing the respective ratio shown above by the deviation of cyclophilin B from the mean value of all housekeeping genes. The results of such quantitative RT-PCR analysis for the SCN2B gene are shown in Figures 3, 4, and 5.

(v) Immunohistochemistry:

For immunofluorescence staining of SCN2B in human brain, frozen sections were prepared with a cryostat (Leica CM3050S) from post-mortem pre-central gyrus of a donor person and fixed in acetone for 10 min at room temperature. After washing in PBS, the sections were pre-incubated with blocking buffer (10% normal goat serum, 0.2% Triton X-100 in PBS) for 30 min and then incubated with affinity-purified rabbit anti-SCN2B antisera (1:20 to 1:40 diluted in blocking buffer; S-6811, Sigma) overnight at 4°C. After rinsing three times in 0.1% Triton X-100/PBS, the sections were incubated with FITC-conjugated goat anti-rabbit IgG antisera (1:150 diluted in 1% BSA/PBS) for 2 hours at room temperature and then again washed in PBS. Staining of the nuclei was performed by incubation of the sections with 5$\mu$M DAPI in PBS for 3 min (blue signal). In order to block the autofluoresence of lipofuscin in human brain, the sections were treated with 1% Sudan Black B in 70% ethanol for 2-10 min at room temperature and then sequentially dipped in 70% ethanol, destilled water and PBS. The sections were coverslipped with 'Vectrashield' mounting medium (Vector Laboratories, Burlingame, CA) and observed under an inverted microscope (IX81, Olympus Optical). The digital images were captured with the appropriate software (AnalySiS, Olympus Optical).

## Claims

1. A method of diagnosing or prognosticating Alzheimer's disease in a subject, or determining whether a subject is at increased risk of developing Alzheimer's Disease, comprising:

    determining a level and/or an activity of

    (i) a transcription product of the gene coding for SCN2B, and/or
    (ii) a translation product of the gene coding for SCN2B, and/or
    (iii) a fragment of said transcription or translation product,

    in a brain sample from said subject and comparing said level and/or said activity to a reference value representing a known disease or health status, thereby diagnosing or prognosticating Alzheimer's Disease in said subject, or determining whether said subject is at increased risk of developing Alzheimer's Disease if SCN2B is dysregulated.

2. The method of claim 1 wherein the sample is a temporal cortex sample and wherein said subject is at increased risk of developing Alzheimer's Disease if SCN2B is downregulated.

3. Use of an antibody specifically immunoreactive with a translation product of a gene coding for SCN2B, SEQ ID NO. 1, or a fragment thereof, for detecting a pathological state of a cell in a brain sample from a subject, comprising immunocytochemical staining of said cell, wherein an altered degree of staining in said cell compared to a cell representing a known health status indicates a pathological state of said cell, and wherein said pathological state relates to Alzheimer's Disease.

## Patentansprüche

1. Verfahren zum Diagnostizieren oder Prognostizieren der Alzheimer-Krankheit bei einem Patienten oder zum Bestimmen, ob ein Patient ein erhöhtes Risiko hat, Alzheimer-Krankheit zu entwickeln, umfassend das Bestimmen einer Konzentration und/oder Aktivität von:

    (i) einem Transcriptionsprodukt des Gens, das für SCN2B codiert; und/oder
    (ii) einem Translationsprodukt des Gens, das für SCN2B codiert; und/oder
    (iii) einem Fragment des Transcriptions- oder Translationsprodukts;

    in einer Gehirnprobe von dem Patienten und Vergleichen der Konzentration und/oder Aktivität mit einem Referenzwert, der einen bekannten Krankheits- oder Gesundheitszustand repräsentiert, wodurch die Alzheimer-Krankheit bei dem Patienten diagnostiziert oder prognostiziert wird oder bestimmt wird, ob der Patient ein erhöhtes Risiko hat, Alzheimer-Krankheit zu entwickeln, wenn SCN2B dysreguliert ist.

2. Verfahren gemäß Anspruch 1, wobei die Probe eine Probe aus dem temporalen Kortex ist und wobei der Patient ein erhöhtes Risiko hat, Alzheimer-Krankheit zu entwickeln, wenn SCN2B herunterreguliert ist.

3. Verwendung eines Antikörpers, der gegenüber einem Translationsprodukt eines Gens, das für SCN2B, SEQ ID Nr. 1 oder ein Fragment davon codiert, spezifisch immunreaktiv ist, zum Nachweis eines pathologischen Zustands einer Zelle in einer Gehirnprobe von einem Patienten, der das immunocytochemische Anfärben der Zelle umfasst, wobei ein veränderter Grad der Anfärbung in der Zelle im Vergleich zu einer Zelle, die einen bekannten Gesundheitszustand repräsentiert, einen pathologischen Zustand der Zelle anzeigt und wobei sich der pathologische Zustand auf die Alzheimer-Krankheit bezieht.

**Revendications**

1. Procédé consistant à établir un diagnostic ou un pré-diagnostic de la maladie d'Alzheimer chez un sujet, ou à déterminer si un sujet présente un risque accru de développement de la maladie d'Alzheimer, procédé comprenant :

   la détermination d'un degré et/ou d'une activité

   (i) d'un produit de transcription du gène codant pour le SCN2B, et/ou
   (ii) d'un produit de traduction du gène codant pour le SCN2B, et/ou
   (iii) d'un fragment dudit produit de transcription ou de traduction,

   dans un échantillon de cerveau provenant dudit sujet et à comparer ledit degré et/ou ladite activité à une valeur de référence représentant une maladie connue ou un état de santé connu, établissant de ce fait un diagnostic ou un pré-diagnostic de la maladie d'Alzheimer chez ledit sujet, ou à déterminer si ledit sujet présente un risque accru de développer la maladie d'Alzheimer si le SCN2B est déréglé.

2. Procédé selon la revendication 1 dans lequel l'échantillon est un échantillon de cortex temporal dans lequel ledit sujet présente un risque accru de développer la maladie d'Alzheimer si le SCN2B présente un dérèglement.

3. Utilisation d'un anticorps spécifiquement immunoréactif avec un produit de traduction d'un gène codant pour le SCN2B, SEQ ID N° 1, ou l'un de ses fragments, en vue de détecter un état pathologique d'une cellule d'un échantillon de cerveau provenant d'un sujet, comprenant une coloration immunocytochimique de ladite cellule, dans laquelle un degré altéré de coloration de ladite cellule comparée à une cellule représentant un état de santé connu indique un état pathologique de ladite cellule, et dans laquelle ledit état pathologique se rapporte à la maladie d'Alzheimer.

Fig. 1: Identification of Genes Involved in Alzheimer's Disease Pathology

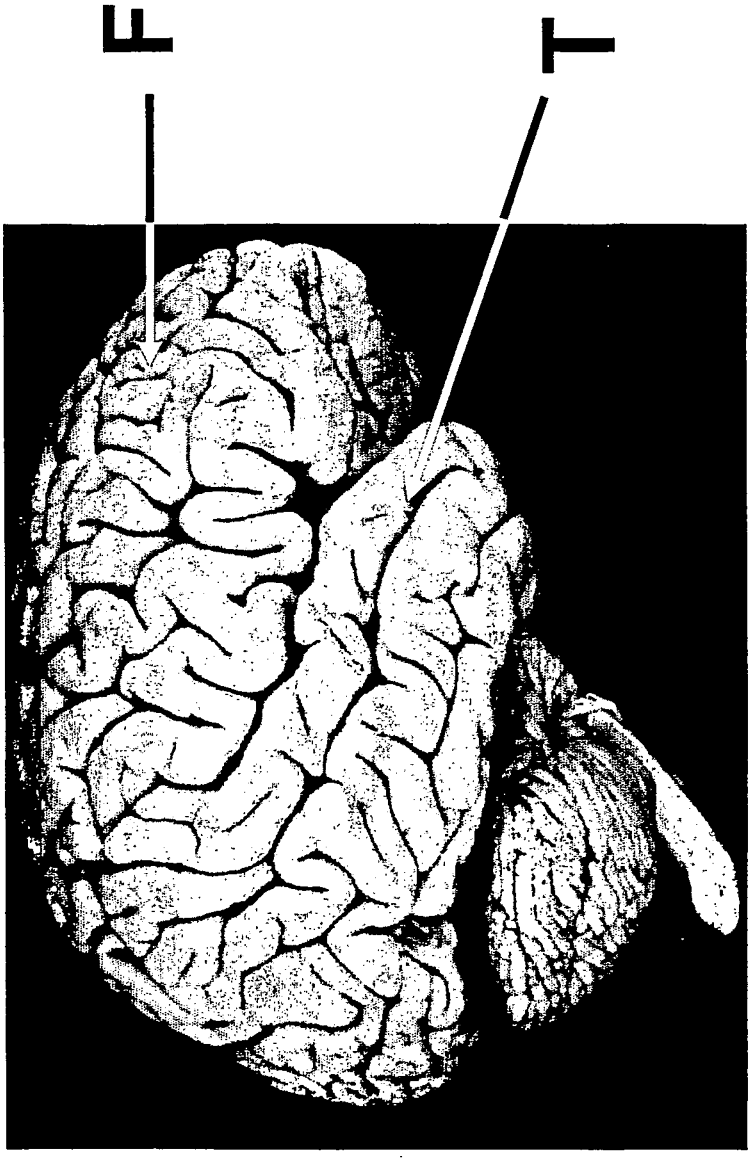

Fig. 2: Identification of differentially expressed genes in a fluorescence differential display screen

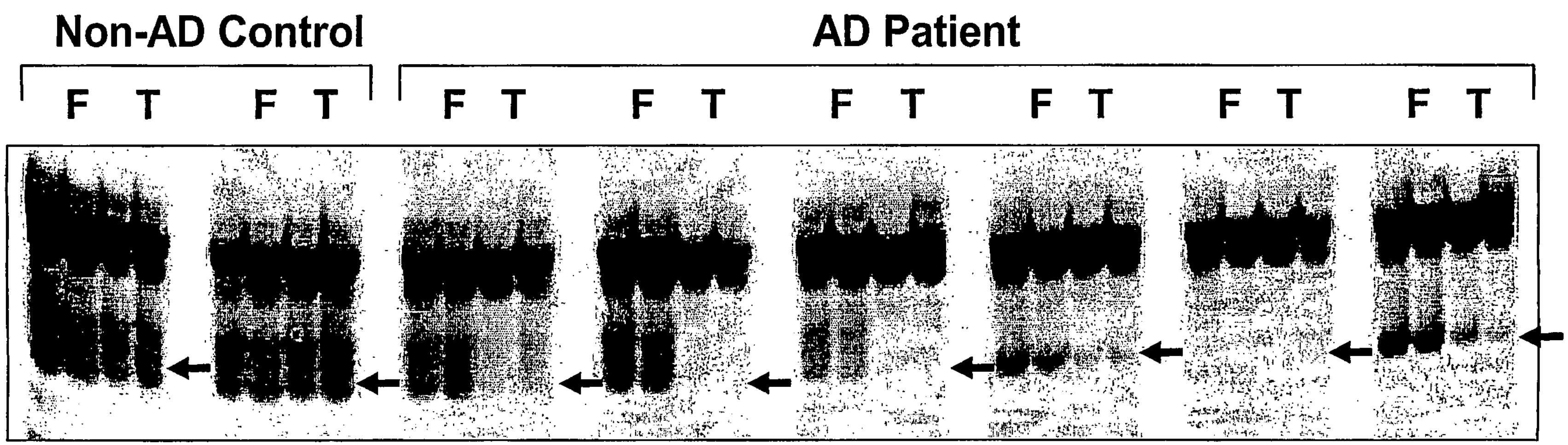

**Fig. 3: Verification of differential expression of SCN2B by quantitative RT-PCR**

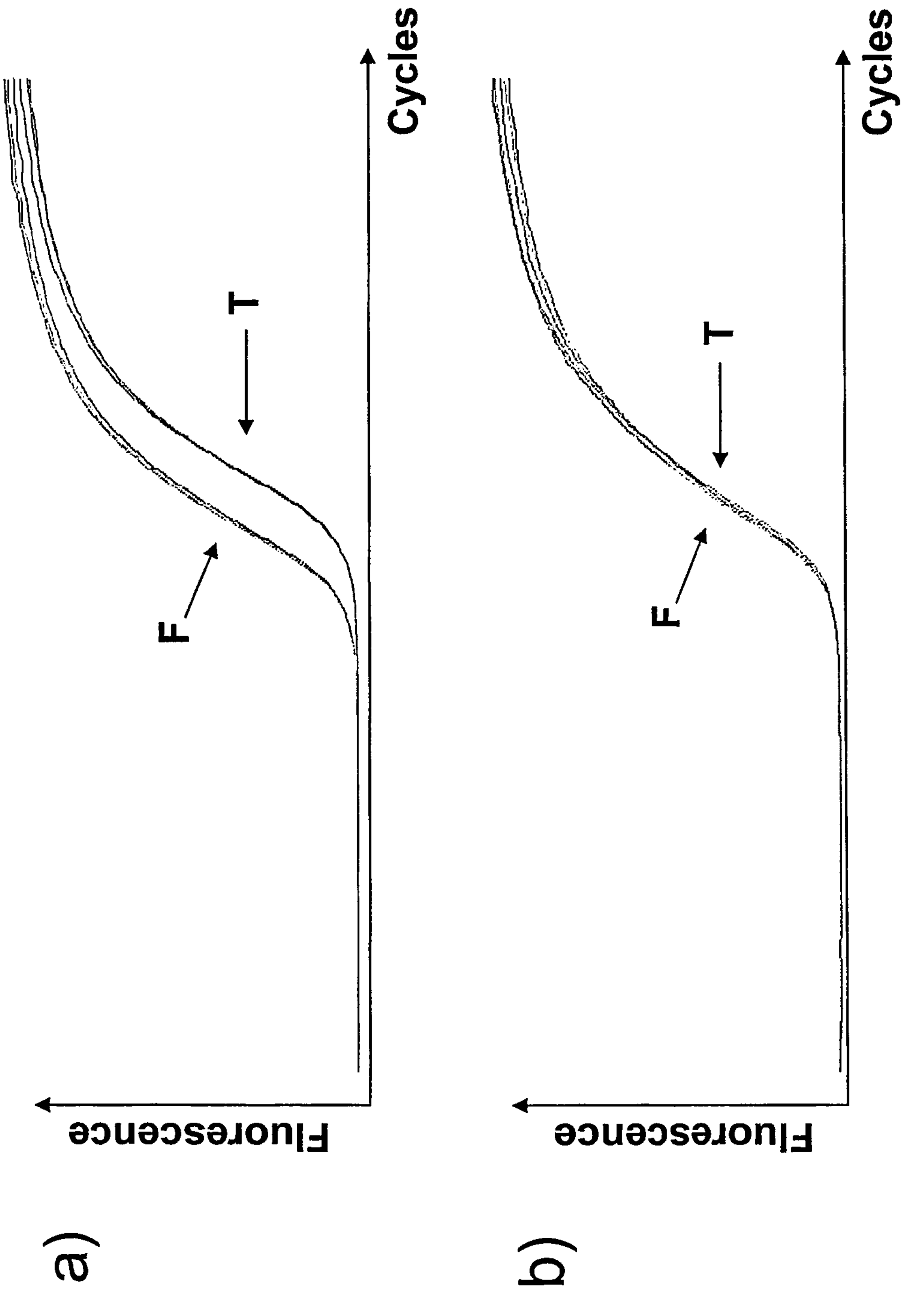

**Fig. 4: Verification of differential expression of SCN2B by quantitative RT-PCR**

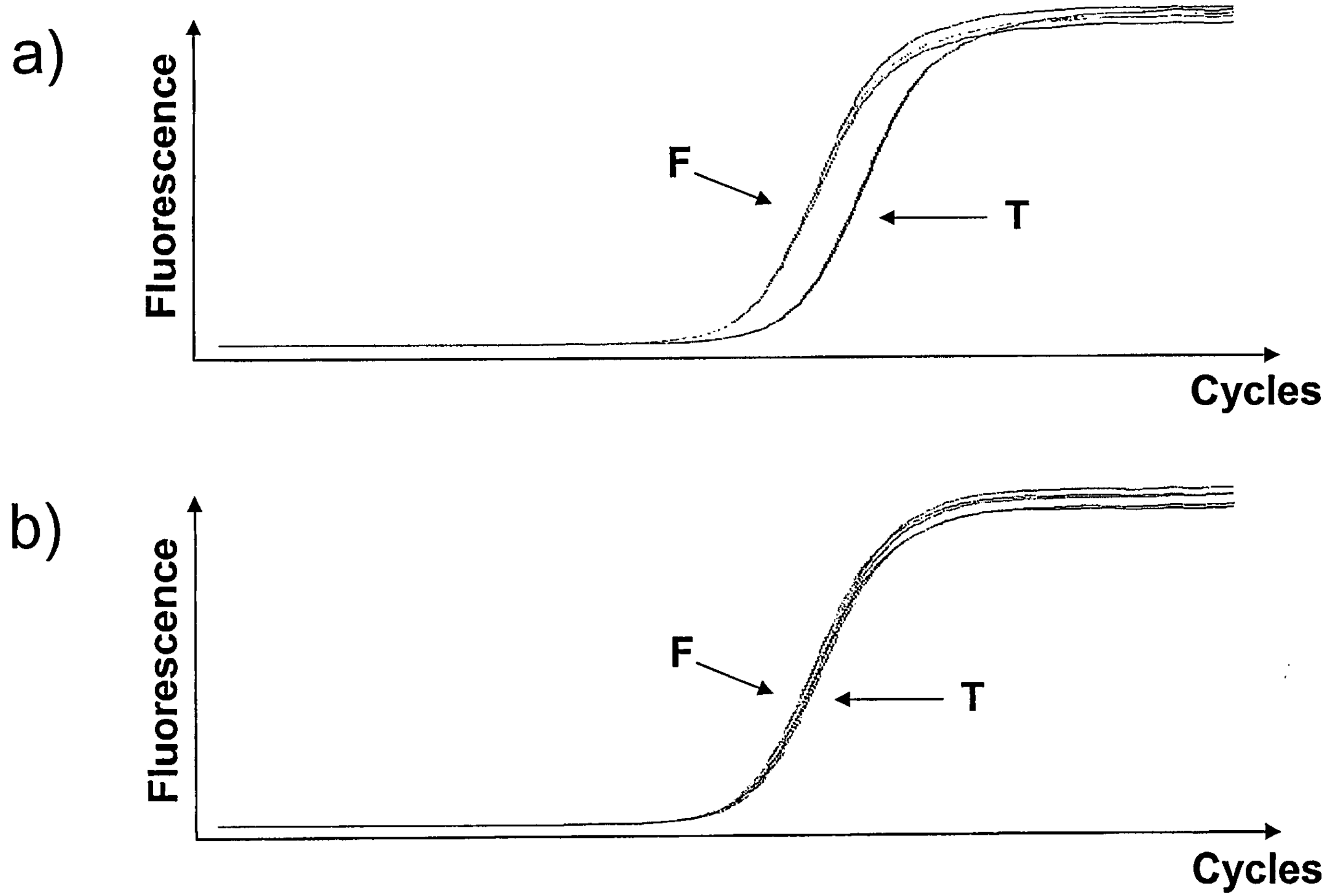

**Fig. 5: Verification of differential expression of SCN2B by quantitative RT-PCR**

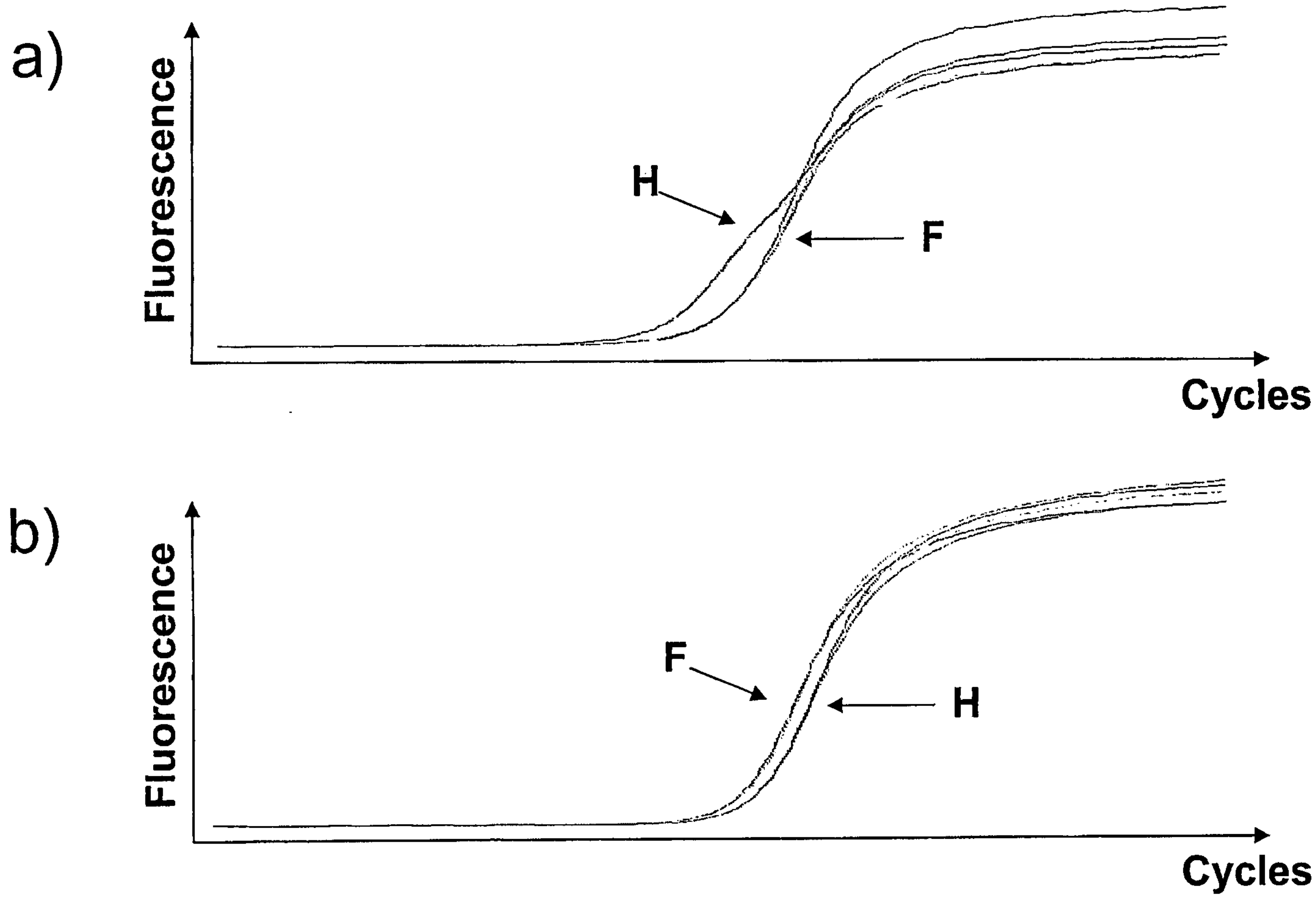

# Fig. 6 : SEQ ID NO. 1:
## amino acid sequence of human SCN2B protein

**Length: 215 aa**

```
  1   MHRDAWLPRP AFSLTGLSLF FSLVPPGRSM EVTVPATLNV LNGSDARLPC
 51   TFNSCYTVNH KQFSLNWTYQ ECNNCSEEMF LQFRMKIINL KLERFQDRVE
101   FSGNPSKYDV SVMLRNVQPE DEGIYNCYIM NPPDRHRGHG KIHLQVLMEE
151   PPERDSTVAV IVGASVGGFL AVVILVLMVV KCVRRKKEQK LSTDDLKTEE
201   EGKTDGEGNP DDGAK
```

# Fig. 7: SEQ ID NO. 2: nucleotide sequence of human SCN2B cDNA

**Length: 4903 bp**

```
   1 CAAGCCGGGC CATATTAGAG AGATGGAAAT AAAGCTTCCT TAATGTTGTA
  51 TATGTCTTTG AAGTACATCC GTGCATTTTT TTTTAGCATC CAACCATTCC
 101 TCCCTTGTAG TTCTCGCCCC CTCAAATCAC CCTCTCCCGT AGCCCACCCG
 151 ACTAACATCT CAGTCTCTGA AAATGCACAG AGATGCCTGG CTACCTCGCC
 201 CTGCCTTCAG CCTCACGGGG CTCAGTCTCT TTTTCTCTTT GGTGCCACCA
 251 GGACGGAGCA TGGAGGTCAC AGTACCTGCC ACCCTCAACG TCCTCAATGG
 301 CTCTGACGCC CGCCTGCCCT GCACCTTCAA CTCCTGCTAC ACAGTGAACC
 351 ACAAACAGTT CTCCCTGAAC TGGACTTACC AGGAGTGCAA CAACTGCTCT
 401 GAGGAGATGT TCCTCCAGTT CCGCATGAAG ATCATTAACC TGAAGCTGGA
 451 GCGGTTTCAA GACCGCGTGG AGTTCTCAGG GAACCCCAGC AAGTACGATG
 501 TGTCGGTGAT GCTGAGAAAC GTGCAGCCGG AGGATGAGGG GATTTACAAC
 551 TGCTACATCA TGAACCCCCC TGACCGCCAC CGTGGCCATG GCAAGATCCA
 601 TCTGCAGGTC CTCATGGAAG AGCCCCCTGA GCGGGACTCC ACGGTGGCCG
 651 TGATTGTGGG TGCCTCCGTC GGGGGCTTCC TGGCTGTGGT CATCTTGGTG
 701 CTGATGGTGG TCAAGTGTGT GAGGAGAAAA AAAGAGCAGA AGCTGAGCAC
 751 AGATGACCTG AAGACCGAGG AGGAGGGCAA GACGGACGGT GAAGGCAACC
 801 CGGATGATGG CGCCAAGTAG TGGGTGGCCG GCCCTGCAGC CTCCCGTGTC
 851 CCGTCTCCTC CCCTCTCCGC CCTGTACAGT GACCCTGCCT GCTCGCTCTT
 901 GGTGTGCTTC CCGTGACCTA GGACCCCAGG GCCCACCTGG GGCCTCCTGA
 951 ACCCCCGACT TCGTATCTCC CACCCTGCAC CAAGAGTGAC CCACTCTCTT
1001 CCATCCGAGA AACCTGCCAT GCTCTGGGAC GTGTGGGCCC TGGGGAGAGG
1051 AGAGAAAGGG CTCCCACCTG CCAGTCCCTG GGGGGAGGCA GGAGGCACAT
1101 GTGAGGGTCC CCAGAGAGAA GGGAGTGGGT GGGCAGGGGT AGAGGAGGGG
1151 GCCGCTGTCC ACCTGCCCAG TGCTTGGCCT GGCAGTGGCT TCAGAGAGGA
1201 CCTGGTGGGG AGGGAGGGCT TTCCTGTGCT GACAGCGCTC CCTCAGGAGG
1251 GCCTTGGCCT GGCACGGCTG TGCTCCTCCC CTGCTCCCAG CCCAGAGCAG
1301 CCATCAGGCT GGAGGTGACG ATGAGTTCCT GAAACTTGGA GGGGCATGTT
1351 AAAGGGATGA CTGTGCATTC CAGGGCACTG ACGGAAAGCC AGGGCTGCAG
1401 GCAAAGCTGG ACATGTGCCC TGGCCCAGGA GGCCATGTTG GGCCCTCGTT
1451 TCCATTGCTA GTGGCCTCCT TGGGGCTCCT GTTGGCTCCT AATCCCTTAG
1501 GACTGTGGAT GAGGCCAGAC TGGAAGAGCA GCTCCAGGTA GGGGGCCATG
1551 TTTCCCAGCG GGGACCCACC AACAGAGGCC AGTTTCAAAG TCAGCTGAGG
1601 GGCTGAGGGG TGGGGCTCCA TGGTGAATGC AGGTTGCTGC AGGCTCTGCC
1651 TTCTCCATGG GGTAACCACC CTCGCCTGGG CAGGGGCAGC CAAGGCTGGG
1701 AAATGAGGAG GCCATGCACA GGGTGGGGCA GCTTTCTTTG GGGCTTCAGT
1751 GAGAACTCTC CCAGTTGCCC TTGGTGGGGT TTCCACCTGG CTTTTGGCTA
1801 CAGAGAGGGA AGGGAAAGCC TGAGGCCGGC ATAAGGGGAG GCCTTGGAAC
1851 CTGAGCTGCC AATGCCAGCC CTGTCCCATC TGCGGCCACG CTACTCGCTC
1901 CTCTCCCAAC AACTCCCTTC GTGGGGACAA AAGTGACAAT TGTAGGCCAG
1951 GCACAGTGGC TCACGCCTGT AATCCCAGCA CTTTGGGAGG CCAAGGCGGG
2001 TGGATTACCT CCATCTGTTT AGTAGAAATG GGCAAAACCC CATCTCTACT
2051 AAAAATACAA GAATTAGCTG GGCGTGGTGG CGTGTGCCTG TAATCCCAGC
2101 TATTTGGGAG GCTGAGGCAG GAGAATCGCT TGAGCCCGGG AAGCAGAGGT
2151 TGCAGTGAAC TGAGATAGTG ATAGTGCCAC TGCAATTCAG CCTGGGTGAC
2201 ATAGAGAGAC TCCATCTCAA AAAAAAAAAA AAAAAAAAAA GGCAATTGTG
```

2251 CGGAGCCAAC CATCACTGCC ATTACACTCA CATTCAATGG AAATTGATTA
2301 GGCCTGAGAG AAGGAGCAAG AGGCAGAAAC ACTGACTCTG AGACCATGAC
2351 CTTGGAAGAT CCTCAGAGAT CAACTAGCCA AGTCACTCAC TTAAAATGGG
2401 GAAACTGAGG TTCCAACTGA CAGAAACTGA GGGTCCTGCC CTCTTCTCTG
2451 GCTCCTCAGT CTCCAGTTTG AAGCAACAGA GCCATCATTT TCCCAGTGGG
2501 AGGGGCCTCT GTGTCCTGGT CTATGATGGG GCGAGTTGGA GGGGTGGGCT
2551 TCTCTAGCCC TGCCCCCTCC CAGGAGCCCA GCCCCTCTGG CCAGGACTGA
2601 GGAGGAGTGG AGGGAAGCAG GGAGGGAGGG AGCAAGTGTG AAGGGCACAG
2651 CTGCTGGCAA ATGCCCCATT CCCTGCCCCA GCACTTCCCA GCCTTTCCAG
2701 GAGCTCTGCC CCACCCCTAC TCAAGGAGTC AGGTGCCCAG GGTAGGGAAT
2751 AAGTCTAGGA AAGGGGGTGG AGACAGGACC GTGACAGTCT CAGGGACCAA
2801 TCCCTGCCTC TGCCCATCAG ATTGCCAATT TGGGGAGACA GCAATATCAT
2851 CTGGGAGATT CAGCTTAATT TGCTTCAATT CAGCAACCAT TTATTGAGCA
2901 TCTATTATGT GCCAGGCACT GTGCCGGGCA CTGGGGATAT ACACAAAGCC
2951 ATAAGCACTG GGGACTGCCA GGTTGGCACT GTAGGGACCA AGATTAACTT
3001 TTTGGTGCAT CTAATTCCAC CTCTGCAAGA CACTGCTTCC AAGGCTACAG
3051 TTGGGGAAGA TGAAGAGATT CTAGAATCTG TGCCCTGGGA AGGGAGAAAC
3101 AGCAGGGAAG AGGGGCAATA CAACAGGGTC AAGGGTGCAG AGGCCAGGCA
3151 GAGTGGGTGC AGAGTGGGGG ATGGGCAACC AGCCTGGTAA CACCTGGGTG
3201 AGGTGAGCGA GTCAAGCCCA TCTGGAAATG CTAAACTCAT CAGCTCAGTA
3251 CCTAACTGCC AATCAACACA CTGCCAATGC GGGTGGGCTA AGCCGCCAGG
3301 ATGCCTGCAG CAGATGGACG CTGCCCATCC TTGAGCCAGC CAGCTGGGGG
3351 CAGAGAGGTG GGTGGCGGGC ACCTCATGCC AGCTGAGACC AAGAGTGGTT
3401 GTATACATTT CTGCAGCTTG CAGATTTCAT TTTTTTGGTC ACCCTTTAAG
3451 TGTCCTTCTC TCCCAGGATG AGTGAGGAGC TGTGCTGATG TTGGGAACTT
3501 ATCTTTTTCC AGGATCCATA ACATTTAGGG GAATTATTTG CGGGCTTTCC
3551 TAGTGTATTC TAAGAAGTTC TAACTCTCCA TCAAGAAGCC CAAGATTCCT
3601 GGAGTCTTTT TTTCCTCTGC CCTGATCAAC TGGGGCCCAG GAAAAGCAAT
3651 GTCTCTTCAC GGGCCTCAGG GCCCCAGCAG ATGGGGCTGG GTCAGGAACC
3701 ATCTCTGTCC CAGGTGCTCA GCCCTCATAT GCATTTTCTT CTGGAGCCTC
3751 TTAGAATCTC CTCCAGAGGA CTCATAGAGC TGCCTCCCCC AGGATGGTGA
3801 AGTTGGGAGC AAAATGGGGC TTCTGTAAGT AGAGGTGGAA AACCAGAGAG
3851 ATGTTTCCAC AGCAGCCATT TCCCTACCCT GTTTCCTAAC TTATGCTGAC
3901 AAGTTCTTCC TCTTCCTACC GTGGGGTTCG AGCCCCACTT CTGGTTGCAT
3951 GTTCCCACCA CCACGCTCTG CTGGTTTTAG TCGGCTGACT TTCTCAACTG
4001 CCCCAGCCTC TCCTGCTCTC TCAGAACCTC CCTCTGCCCC CACCCCCCAT
4051 CTGTCTCTTC CTCCTAATTA AGGGCCACTG CAAAGAGAGA CGGGCTGGCT
4101 AACCCATGGG ATTTAGCTTA TAAGAACAAA GCACAATTAT TCAGGACTGA
4151 ACTGCTTTGA GGTGCCTTGG AACTGGCTTT GGAAATAGCT ATCTGCCCCC
4201 TAGCTATGGA CTCTTCACAG TTATTTCCAG TTTATAAAAA GGAAAAGCTG
4251 ATCGGATCCA TGTTTCTCTC TGCGAGTCTC CTTGGGGAGG TTGGGCTGGT
4301 AAATATTTGC AAGGTCAGGT TAAGCAGACA GACAATTGAA AATACTGGGC
4351 AGGTCCAAAA GGATAGTTCC AGGCCATAGG AATGGCTTGG TGCAGTTAAC
4401 GGGTGATGAA GCAAGAAATC TTCCTTCCTA CTTATGTCTT AAAGATGGAC
4451 AGCCTGTGTC TCTTGCTGTG CCTCTTGCTG GCGTCTTCCT TTATTGAAGC
4501 TTGCTGATGC ACTGTACATA GTCTCTCTCT CTAGGTATAG ATATATTATA
4551 TATACAAATA TCAAACATCT CACCACACCC ACCCCAAAAG TTACACTGTG
4601 TGTGTGTGGG GAGGTGTAAT AACTTCTCAG TGTTTTGAGT TGATTCACCA
4651 AAGGCAAATT TTGGAATGTT CTTGCGCTTT GTGCACTTGG AGGGGAAAAC
4701 CCAACTACAT ATGCACTTTC TTGGTTGTGT GTGCTATAGA GAGGTTGGAA
4751 GGGGTGGTTG GAAGGGGAGA GAAACTGAGA TGGCCCTCTG TGACTCGACT
4801 GCTGGGATGT ATCTGCTTTT GGGAGCAGAC TGAGTTTCTT TTGCAATTTG
4851 TCTTATTGTT TGGGTCTACT GGCGACAATA AACTATCTCC CCTGAAAAAA
4901 AAA

## Fig. 8: SEQ ID NO. 3

**Length: 99 bp**

1 CAGGGGAGGT AGTTTATTGT CGCCAGTAGA CCCAAACAAT AAGACAAATT

51 GCAAAAGAAA CTCAGTCTGC TCCCAAAAGC AGATACATCC CAGCAGTCG

# Fig. 9: Alignment of SEQ ID NO. 3, with the human SCN2B cDNA

**Length: 99 bp**

```
                 .         .         .         .         .
  99 CGACTGCTGGGATGTATCTGCTTTTGGGAGCAGACTGAGTTTCTTTTGCA 50
     ||||||||||||||||||||||||||||||||||||||||||||||||||
4796 CGACTGCTGGGATGTATCTGCTTTTGGGAGCAGACTGAGTTTCTTTTGCA 4845
                 .         .         .         .
  49 ATTTGTCTTATTGTTTGGGTCTACTGGCGACAATAAACTACCTCCCCTG 1
     |||||||||||||||||||||||||||||||||||||||| ||||||||
4846 ATTTGTCTTATTGTTTGGGTCTACTGGCGACAATAAACTATCTCCCCTG 4894
```

**Fig. 10: Schematic assembly of SEQ ID NO. 2, with human mRNAs (AF049498, AF107028, AX376000, HSU87555) and human ESTs**

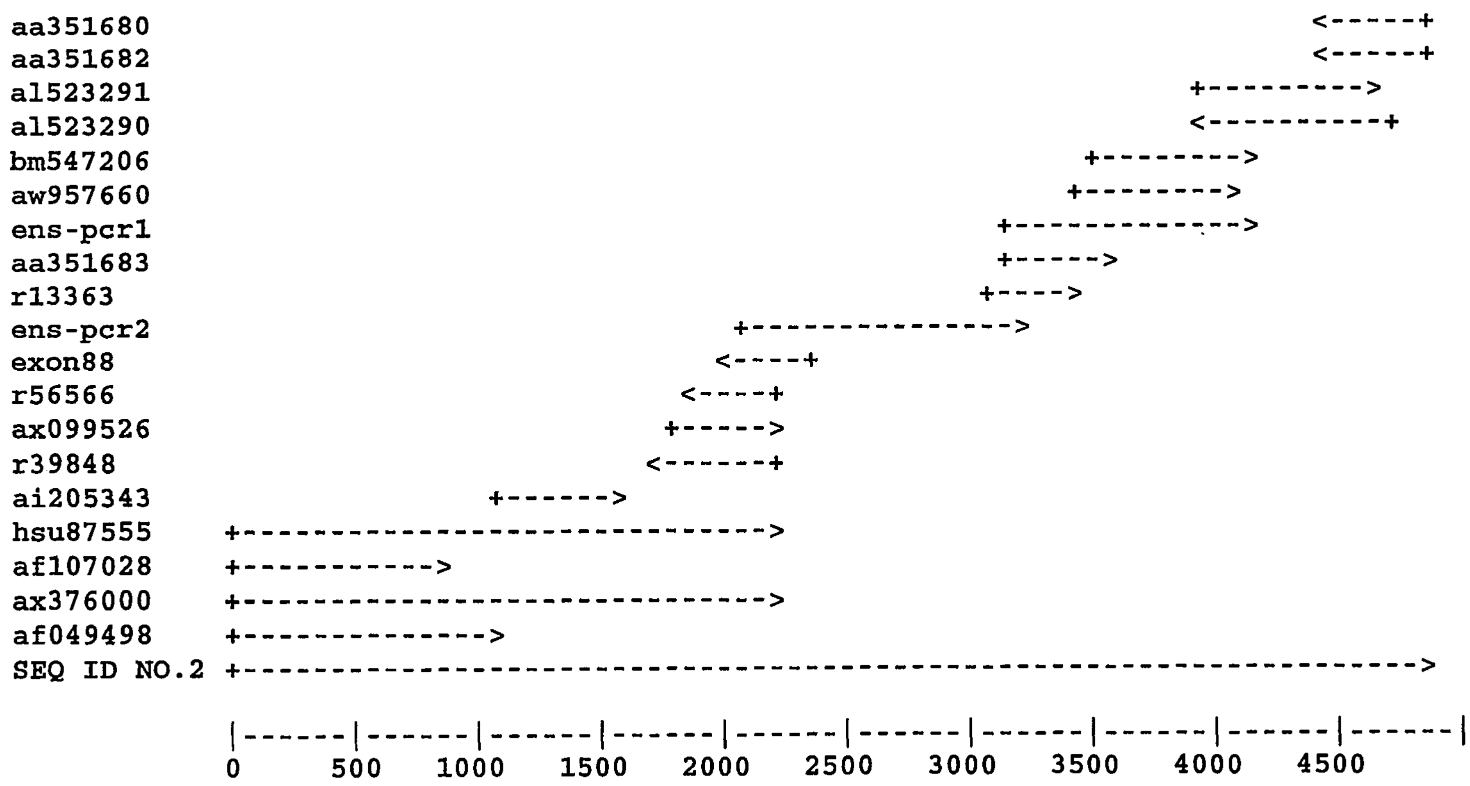

**Fig. 11: Schematic alignment of SEQ ID NO. 3 with human SCN2B cDNA, SEQ ID NO. 2**

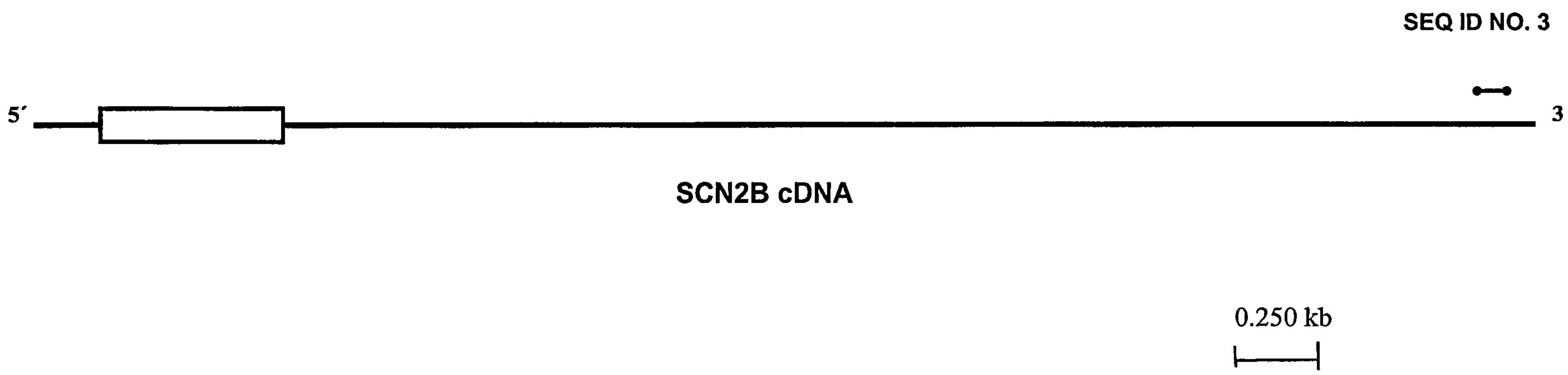

**Fig. 12: Images of human pre-central brain sections labeled with anti-SCN2B antiserum and with DAPI**

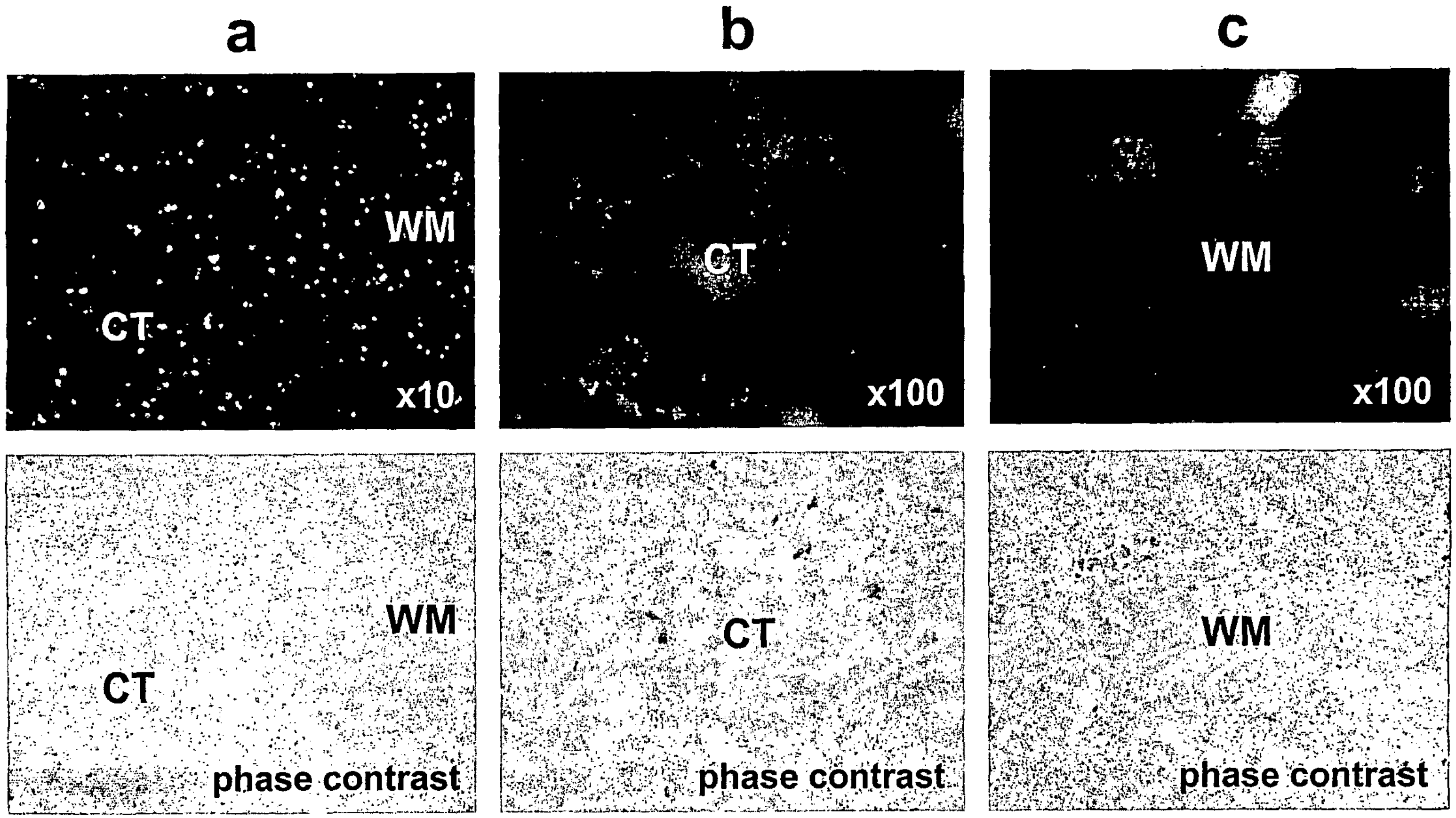

# Fig.13

| sample | | Δ (fold) (temporal/ frontal cortex) |
|---|---|---|
| control | C011 | 0.59 |
| control | C012 | 0.85 |
| control | C014 | 2.15 |
| control | C005 | 0.90 |
| control | C008 | 1.01 |
| control | C020 | 0.91 |
| control | C022 | 0.66 |
| control | C024 | 0.98 |
| control | C025 | 1.17 |
| control | C026 | 0.68 |
| control | C027 | 1.00 |
| control | C028 | 1.03 |
| control | C029 | 0.84 |
| control | C030 | 0.75 |
| control | C031 | 0.73 |
| control | C032 | 0.86 |
| control | C033 | 1.32 |
| control | C034 | 0.86 |
| control | C035 | 0.65 |
| patient | P012 | 0.62 |
| patient | P016 | 0.61 |
| patient | P010 | 0.29 |
| patient | P011 | 0.72 |
| patient | P014 | 0.61 |
| patient | P017 | 0.98 |
| patient | P019 | 0.63 |
| patient | P026 | 0.79 |
| patient | P031 | 0.65 |
| patient | P038 | 0.71 |
| patient | P040 | 0.44 |
| patient | P041 | 0.69 |
| patient | P042 | 0.62 |
| patient | P046 | 0.40 |
| patient | P047 | 0.69 |

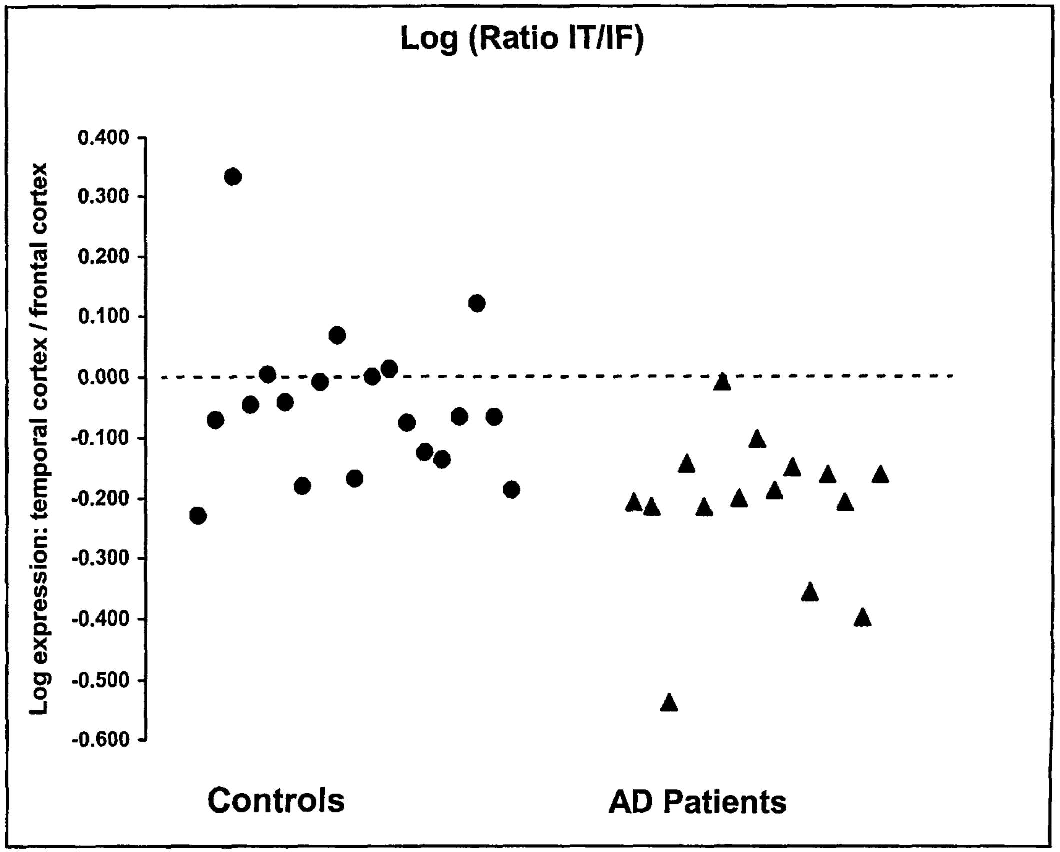

Fig.14 :

| sample | | Δ (fold) (temporal/ frontal cortex) |
|---|---|---|
| control | C011 | 0.63 |
| control | C012 | 0.48 |
| control | C014 | 1.48 |
| control | C005 | 0.75 |
| control | C008 | 0.51 |
| patient | P012 | 0.29 |
| patient | P016 | 0.36 |
| patient | P010 | 0.91 |
| patient | P011 | 0.38 |
| patient | P014 | 0.56 |
| patient | P017 | 0.36 |
| patient | P019 | 0.20 |

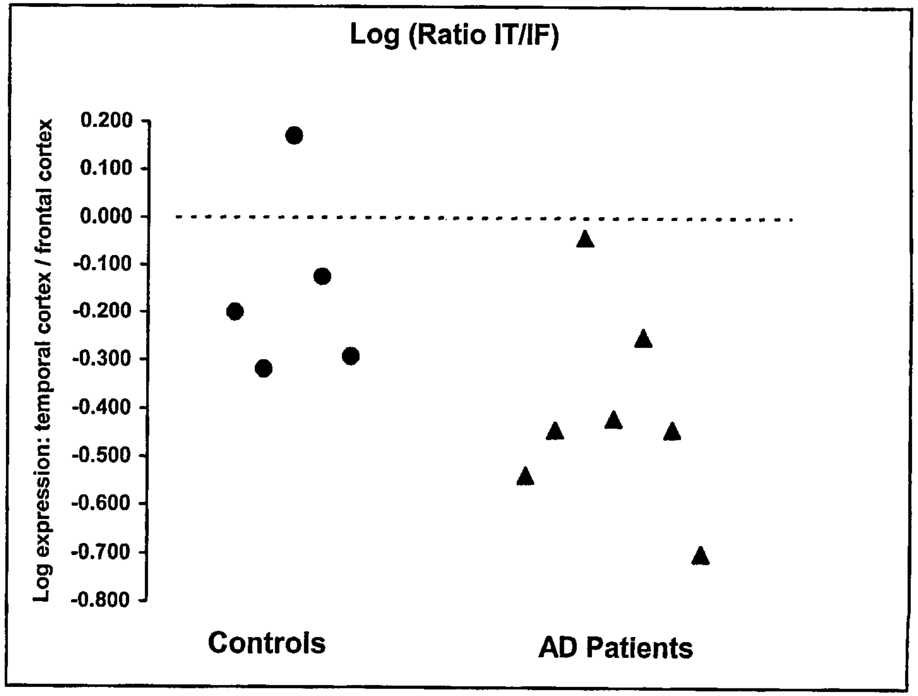

# Fig.15

| sample | | Δ (fold) (hippocampus / frontal cortex) |
|---|---|---|
| control | C005 | 0.77 |
| control | C008 | 1.26 |
| control | C004 | 0.98 |
| patient | P012 | 1.24 |
| patient | P016 | 0.61 |
| patient | P010 | 0.48 |
| patient | P011 | 10.87 |
| patient | P014 | 1.37 |
| patient | P019 | 0.80 |

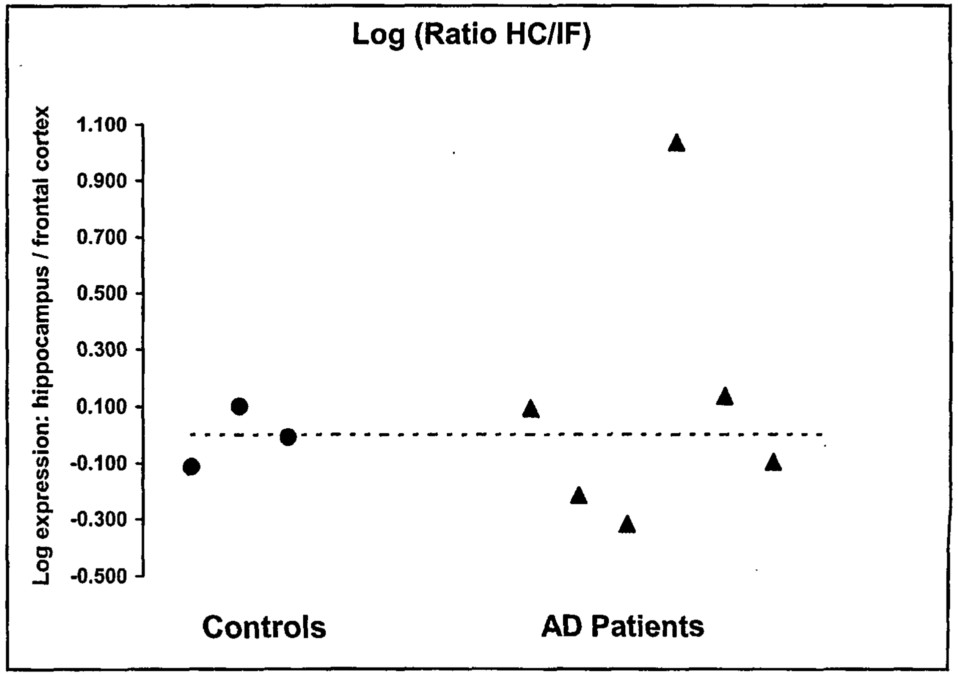

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0219966 A
- WO 02087419 A
- WO 02090532 A
- WO 0179547 A
- WO 0214543 A
- US 6150173 A


### Non-patent literature cited in the description


- **VICKERS et al.** *Progress in Neurobiology,* 2000, vol. 60, 139-165
- **SELKOE.** *Physiological Rev,* 2001, vol. 81, 741-66
- **GREENFIELD et al.** *Frontiers Bioscience,* 2000, vol. 5, D72-83
- **BRAAK ; BRAAK.** *Acta Neuropathol,* 1991, vol. 82, 239-259
- **JOHNSON ; JENKINS.** *J Alzheimers Dis,* 1996, vol. 1, 38-58
- **JOHNSON ; HARTIGAN.** *J Alzheimers Dis,* 1999, vol. 1, 329-351
- **SCHMITT et al.** *Neurology,* 2000, vol. 55, 370-376
- **TERRY et al.** *Annals of Neurology,* 1981, vol. 10, 184-92
- **STRITTMATTER et al.** *Proc Natl Acad Sci USA,* 1993, vol. 90, 1977-81
- **ROSES.** *Ann NY Acad Sci,* 1998, vol. 855, 738-43
- **MYERS et al.** *Science,* 2000, vol. 290, 2304-5
- **BERTRAM et al.** *Science,* 2000, vol. 290, 2303
- **SCOTT et al.** *Am J Hum Genet,* 2000, vol. 66, 922-32
- **LEHMANN-HORN et al.** *Physiological Reviews,* 1999, vol. 79, 1317-1358
- **NODA et al.** *Nature,* 1986, vol. 322, 826-828
- **SCHALLER et al.** *Journal of Neuroscience,* 1995, vol. 15, 3231-3242
- **ISOM et al.** *Neuron,* 1994, vol. 12, 1183-1194
- **ISOM et al.** *Cell,* 1995, vol. 83, 443-445
- **MORGAN et al.** *Proceedings National Academy of Science USA,* 2000, vol. 97, 2308-2313
- **PATTON et al.** *Journal Biological Chemistry,* 1994, vol. 269, 17649-17655
- **EUBANKS et al.** *Neuroreport,* 1997, vol. 8, 2775-2779
- **BOLINO et al.** *European Journal of Human Genetics,* 1998, vol. 6, 629-634
- **HAUG et al.** *Neuroreport,* 2000, vol. 11, 2687-2689
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **DEVEREUX et al.** *Nucleic Acids Res.,* 1984, vol. 12, 387
- **PEARSON ; LIPMAN.** *Proc. Natl. Acad. Sci. USA,* 1988, vol. 85, 2444-2448
- **WILBUR ; LIPMAN.** *SIAM J. Appl. Math.,* 1984, vol. 44, 557-567
- **NEEDLEMAN ; WUNSCH.** *J. Mol. Biol.,* 1970, vol. 48, 443-453
- **SAMBROOK ; RUSSELL.** Molecular Cloning: A Laboratory Manual. Cold Spring Harbor Laboratory Press, 2001
- **SCHENA M.** Microarray Biochip Technology. Eaton Publishing, 2000
- **HARLOW ; LANE.** Using Antibodies: A Laboratory Manual. Cold Spring Harbor Laboratory Press, 1999
- **EDWARDS R.** Immunodiagnostics: A Practical Approach. Oxford University Press, 1999
- **HARLOW et al.** Antibodies: A Laboratory Manual. Cold Spring Harbor Laboratory Press, 1988
- **DUBEL ; BREITLING.** Recombinant Antibodies. Wiley-Liss, 1999
- **EDWARDS R.** Immunodiagnostics: A Practical Approach. Oxford University Press, 1999
- **TERRY et al.** *Annals of Neurology,* 1981, vol. 10, 184-192
- **STRANGE.** Brain Biochemistry and Brain Disorders. Oxford University Press, 4
- **LIANG ; PARDEE.** *Science,* 1995, vol. 267, 1186-7
- **VON DER KAMMER et al.** *Nucleic Acids Research,* 1999, vol. 27, 2211-2218
- **LIANG et al.** *Nucleic Acids Research,* 1994, vol. 22, 5763-5764
- **ZHAO et al.** *Biotechniques,* 1995, vol. 18, 842-850